# EUROPEAN PATENT APPLICATION

(11) **EP 4 159 278 A1**
(43) Date of publication of application: **05.04.2023**
(21) Application number: 21812719.9
(22) Date of filing: 14.04.2021
(51) Int. Cl.: A61Q 1/04, A61Q 1/10, A61Q 15/00, A61Q 17/04, A61Q 19/00, A61Q 19/10, A61Q 5/00, A61K 8/891

(54) **COSMETIC PREPARATAION**

(30) Priority: 26.05.2020 JP 2020091528
(71) Applicant: SHIN-ETSU CHEMICAL CO., LTD., Tokyo 1000005 (JP)
(72) Inventor: KIKUCHI Hiroko, Tokyo (JP); MIZUNO Akiko, Tokyo (JP); YANAGIDA Sachie, Tokyo (JP); KANAI Tomoya, Annaka-shi, Gunma 379-0224 (JP); ABE Takuya, Annaka-shi, Gunma 379-0224 (JP); HATA Ryunosuke, Annaka-shi, Gunma 379-0224 (JP)
(74) Representative: Sonnenhauser, Thomas Martin
(86) International application number: PCT/JP2021/015476
(87) International publication number: WO 2021/241049

(57) **Abstract**

The present invention is a cosmetic containing at least one organosiloxane represented by the following general formula (1), and having a boiling point in a range of 205 to 255°C and a viscosity of less than 5 mm²/s at 25°C. In the formula, R's are identical to or different from one another and each independently represent a hydrogen group, a hydroxy group, or a monovalent hydrocarbon group having 1 to 3 carbon atoms, but at least one R is a monovalent hydrocarbon group having 2 to 3 carbon atoms. "a" represents 1 to 5. This provides a cosmetic which has light touch, good spread, and excellent water repellency, forms uniform cosmetic film, and successfully achieves favorable feeling on use with no strong oily feeling, and which has excellent stability over time and cosmetic persistence even when blended with various oil agents, such as silicone, hydrocarbon oil, and ester, an organic ultraviolet absorber, or an oily component solid at 25°C.

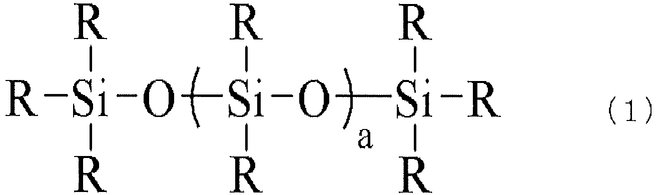

## Description

### TECHNICAL FIELD

The present invention relates to a cosmetic blended with a volatile organosiloxane.

### BACKGROUND ART

Silicones typified by dimethylpolysiloxane have features such as light touch, good spread, excellent water repellency, and high safety, and therefore in recent years, silicones have been often used as oil agents used for cosmetics.

For example, a cosmetic containing a volatile cyclic siloxane such as octamethyltetrasiloxane (D4), decamethylpentasiloxane (D5), or dodecamethylhexasiloxane (D6), a linear siloxane having a viscosity of 0.65 to 6 mm²/second at 25°C, or a volatile siloxane having a branched siloxane chain such as tristrimethylsiloxymethylsilane (M3T) is excellent in a light touch, a good spread, and water repellency when the cosmetic is applied to the skin (Patent Document 1).

However, these silicones have problems that the compatibility with a polar oil agent is poor and the silicones cannot be mixed therewith in a clear state. The feeling of use and stability of the cosmetic may deteriorate. In particular, this trend is clearly observed when an organic ultraviolet absorber is used. Moreover, if silicone has low affinity for an oily component, such as a wax, blended for solidification of a cosmetic, the crystallization of this oily component is hindered, and an expected hardness of a preparation cannot be obtained.

To solve these problems, that is, to enhance the compatibility with a polar oil agent, wax, and so forth, it is known that a phenyl-modified silicone, an ester, and the like are used as compatibilizers. In these cases, however, the feeling on use is heavy, and the light touch of silicone may be lost. Further, most of oil agents such as phenyl-modified silicones and esters are non-volatile or does not have high volatility, so that oily feeling may be strongly sensed after application of the cosmetics to the skin.

Meanwhile, for the purpose of providing an alternative in order to formulate a composition containing a volatile oil, Patent Document 2 proposes a composition which contains at least one non-cyclic volatile silicone oil, and which has a specific evaporation profile. However, this document fails to state formulation embodiments other than decamethyltetrasiloxane and dodecamethylpentasiloxane, and does not disclose any examination of using non-cyclic volatile silicone oil to achieve compatibility improvement with respect to polar oil agent, wax, and so forth.

Meanwhile, Patent Document 3 discloses that the feeling on use of a composition in which some methyl groups of non-cyclic dimethylsiloxane are substituted with ethyl groups is examined. However, this patent document does not examine at all compatibility improvement with respect to polar oil agent, wax, and so forth when the composition is blended into a cosmetic.

### CITATION LIST

### PATENT LITERATURE

Patent Document 1: JP 3658561 B
Patent Document 2: WO 2004/087077 A1
Patent Document 3: US 2010/0144897 A1

### SUMMARY OF INVENTION

### TECHNICAL PROBLEM

The present invention has been made in view of the above circumstances. An object of the present invention is to provide a cosmetic which has light touch, good spread, and excellent water repellency, forms a uniform cosmetic film, and also successfully achieves favorable feeling on use with no strong oily feeling, and which has excellent stability over time and cosmetic persistence even when blended with various oil agents, such as silicone, hydrocarbon oil, and ester, an organic ultraviolet absorber, or an oily component being solid at 25°C.

### SOLUTION TO PROBLEM

To solve the problems, the present invention provides a cosmetic comprising at least one organosiloxane represented by the following general formula (1), and having a boiling point in a range of 205 to 255°C and a viscosity of less than 5 mm²/s at 25°C, 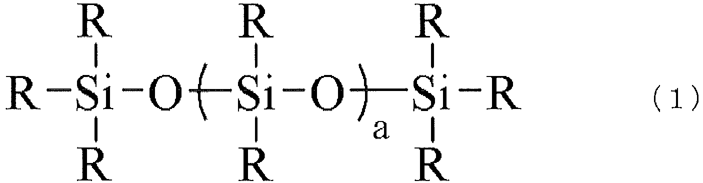 wherein R's are identical to or different from one another and each independently represent a hydrogen group, a hydroxy group, or a monovalent hydrocarbon group having 1 to 3 carbon atoms, but at least one R is a monovalent hydrocarbon group having 2 to 3 carbon atoms; and "a" represents 1 to 5.

Such a cosmetic has light touch, good spread, and excellent water repellency, forms uniform cosmetic film, and also successfully achieves favorable feeling on use without strong oily feeling. Further, this cosmetic has excellent stability over time and cosmetic persistence even when blended with various oil agents, such as silicone, hydrocarbon oil, and ester, an organic ultraviolet absorber, or an oily component being solid at 25°C.

Additionally, in this case, in the general formula (1) of the organosiloxane, "a" is preferably 2.

The cosmetic containing such an organosiloxane does not cause strong oily feeling and can achieve favorable feeling on use. Even when blended with various oil agents, organic ultraviolet absorber, or oily component being solid at 25°C, the cosmetic has excellent stability over time and cosmetic persistence.

Further, the organosiloxane is more preferably selected from the group consisting of 3,5-diethyl-1,1,1,3,5,7,7,7-octamethyltetrasiloxane, 3,3-diethyl-1,1,1,5,5,7,7,7-octamethyltetrasiloxane, 1,3-diethyl-1,1,3,5,5,7,7,7-octamethyltetrasiloxane, 1,5-diethyl-1,1,3,3,5,7,7,7-octamethyltetrasiloxane, 1,7-diethyl-1,1,3,3,5,5,7,7-octamethyltetrasiloxane, 3,5-dipropyl-1,1,1,3,5,7,7,7-octamethyltetrasiloxane, 3,3-dipropyl-1,1,1,5,5,7,7,7-octamethyltetrasiloxane, 1,3-dipropyl-1,1,3,5,5,7,7,7-octamethyltetrasiloxane, 1,5-dipropyl-1,1,3,3,5,7,7,7-octamethyltetrasiloxane, 1,7-dipropyl-1,1,3,3,5,5,7,7-octamethyltetrasiloxane, and 3-ethyl-5-propyl-1,1,1,3,5,7,7,7-octamethyltetrasiloxane.

The cosmetics containing such organosiloxanes can more surely achieve favorable feeling on use without provoking strong oily feeling. Even when blended with various oil agents, organic ultraviolet absorber, or oily component being solid at 25°C, the cosmetics have excellent stability over time and cosmetic persistence.

More preferably, the inventive cosmetic further comprise an organic ultraviolet absorber.

In the present invention, the organosiloxane has excellent compatibility with an organic ultraviolet absorber. Accordingly, the cosmetic containing such an organic ultraviolet absorber has favorable feeling on use, and excellent stability over time and cosmetic persistence.

In this case, the organic ultraviolet absorber preferably comprises one or more selected from ethylhexyl methoxycinnamate, diethylamino hydroxybenzoyl hexyl benzoate, octyl salicylate, polysilicone-15, t-butyl methoxydibenzoylmethane, oxybenzone, methylene bis-benzotriazolyl tetramethylbutylphenol, bis-ethylhexyloxyphenol methoxyphenyl triazine, and octocrylene.

Such organic ultraviolet absorbers are particularly preferable because the effect of absorbing ultraviolet ray is high and the compatibility with the organosiloxane of the present invention is excellent.

More preferably, the inventive cosmetic further comprises an oily component being solid at 25°C.

The organosiloxane in the present invention is preferable because even when an oily component solid at 25°C is used in combination, the affinity for an oily component is so high that a stick-form preparation and so forth can be easily prepared without hindering solidification of the oily component.

In this case, the oily component being solid at 25°C preferably comprises one or more selected from polyethylene, ceresin, ozokerite, beeswax, microcrystalline wax, stearyl alcohol, behenyl alcohol, and cetanol.

Such oily components are particularly preferable because of high affinity for the organosiloxane in the present invention.

### ADVANTAGEOUS EFFECTS OF INVENTION

The inventive cosmetic includes the organosiloxane(s) shown by the general formula (1) as a base composition. This makes the cosmetic have light touch, good spread, and excellent water repellency, form a uniform cosmetic film, and have excellent stability over time and cosmetic persistence even in a mixture system with an organic ultraviolet absorber. In addition, when an oily component solid at 25°C is used in combination, a stick-form preparation and the like can be easily prepared without hindering solidification of this oily component. Moreover, since the organosiloxane(s) of the present invention have a boiling point in a range of 205 to 255°C and are volatile or exhibit high volatility, the cosmetic does not cause strong oily feeling after the application to the skin. Further, since the organosiloxane(s) in the present invention have a viscosity of less than 5 mm²/s (25°C), the organosiloxane(s) do not cause considerable sticky feeling, and the feeling on use is favorable. Furthermore, the present invention does not require exact volatilization-rate control through a combination of an organosiloxane having high volatility and an organosiloxane having mild volatility. To put it differently, the above object is achieved by solely using the organosiloxane of the present invention, that is, organosiloxane of the general formula (1) having a boiling point in a range of 205 to 255°C.

### DESCRIPTION OF EMBODIMENTS

The present inventors have earnestly studied to achieve the above-described object and consequently found that when a cosmetic contains an organosiloxane having a specific structure, and having a boiling point and a viscosity within predetermined ranges, the cosmetic has light touch, good spread, and excellent water repellency, forms uniform cosmetic film, and can also achieve favorable feeling on use with no strong oily feeling. Even when blended with various oil agents, such as silicone, hydrocarbon oil, and ester, an organic ultraviolet absorber, or an oily component being solid at 25°C, this cosmetic has excellent stability over time and cosmetic persistence. This finding has led to the completion of the present invention.

Specifically, the present invention is a cosmetic comprising at least one organosiloxane represented by the following general formula (1), and having a boiling point in a range of 205 to 255°C and a viscosity of less than 5 mm²/s at 25°C, 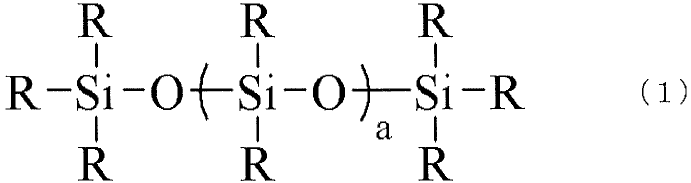 wherein R's are identical to or different from one another and each independently represent a hydrogen group, a hydroxy group, or a monovalent hydrocarbon group having 1 to 3 carbon atoms, but at least one R is a monovalent hydrocarbon group having 2 to 3 carbon atoms; and "a" represents 1 to 5.

Hereinafter, the present invention will be described in detail, but the present invention is not limited thereto.

The inventive cosmetic contains at least one organosiloxane which is represented by the following general formula (1), and which has a boiling point in a range of 205 to 255°C and a viscosity of less than 5 mm²/s (25°C). The lower limit of the viscosity is not particularly limited, and can be more than 0 mm²/s. 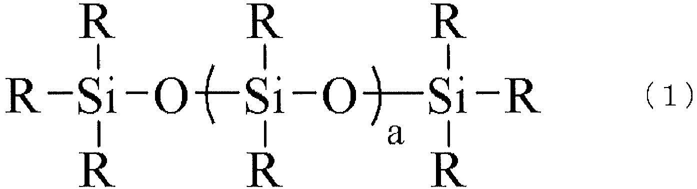 In the formula, R's are identical to or different from one another and each independently represent a hydrogen group, a hydroxy group, or a monovalent hydrocarbon group having 1 to 3 carbon atoms, but at least one R is a monovalent hydrocarbon group having 2 to 3 carbon atoms. "a" represents 1 to 5.

In the general formula (1), each R is identical to or different from the others and is independently a hydrogen group, a hydroxy group, or a monovalent hydrocarbon group having 1 to 3 carbon atoms. Specific examples thereof include a methyl group, an ethyl group, and a propyl group. Note that at least one R is a monovalent hydrocarbon group having 2 to 3 carbon atoms, and specific examples thereof include an ethyl group and a propyl group.

"a" is preferably 2.

Since the boiling point of the organosiloxane in the present invention ranges from 205 to 255°C, the organosiloxane has a volatility equivalent to those of decamethylcyclopentasiloxane (boiling point: 210°C), dodecamethylcyclohexasiloxane (boiling point: 245°C), etc. Hence, the cosmetic applied to skin does not provoke strong oily feeling. If the boiling point is lower than 205°C, it is difficult to favorably apply and spread the cosmetic. If the boiling point is higher than 255°C, strong oily feeling is sensed.

Further, since the viscosity of the organosiloxane in the present invention is less than 5 mm²/s (25°C), favorable applicability and spreadability are sensed in the same manner as in cases of using decamethylcyclopentasiloxane having a viscosity of 4 mm²/s (25°C), or the like. Additionally, the feeling on use is favorable without provoking considerable sticky feeling. If the viscosity is 5 mm²/s (25°C) or higher, this causes poor spreading, sticky feeling is provoked, and the feeling on use is poor. Note that the viscosity (kinematic viscosity) values are measured using an Ostwald viscometer.

The organosiloxane in the present invention is preferably selected from the group consisting of 3,5-diethyl-1,1,1,3,5,7,7,7-octamethyltetrasiloxane, 3,3-diethyl-1,1,1,5,5,7,7,7-octamethyltetrasiloxane, 1,3-diethyl-1,1,3,5,5,7,7,7-octamethyltetrasiloxane, 1,5-diethyl-1,1,3,3,5,7,7,7-octamethyltetrasiloxane, 1,7-diethyl-1,1,3,3,5,5,7,7-octamethyltetrasiloxane, 3,5-dipropyl-1,1,1,3,5,7,7,7-octamethyltetrasiloxane, 3,3-dipropyl-1,1,1,5,5,7,7,7-octamethyltetrasiloxane, 1,3-dipropyl-1,1,3,5,5,7,7,7-octamethyltetrasiloxane, 1,5-dipropyl-1,1,3,3,5,7,7,7-octamethyltetrasiloxane, 1,7-dipropyl-1,1,3,3,5,5,7,7-octamethyltetrasiloxane, and 3-ethyl-5-propyl-1,1,1,3,5,7,7,7-octamethyltetrasiloxane.

Moreover, the organosiloxane in the present invention is excellent in compatibility with general-purpose components blended into various cosmetics. Thus, it is possible to use combinations with any such components. Particularly, the organosiloxane is excellent in compatibility with organic ultraviolet absorbers, such as ethylhexyl methoxycinnamate. Further, in preparation by blending silicone which is adopted to exhibit light feeling on use, the organosiloxane results in a cosmetic excellent in stability over time and cosmetic persistence.

Furthermore, when an oily component being solid at 25°C, such as polyethylene, ceresin, ozokerite, beeswax, microcrystalline wax, stearyl alcohol, behenyl alcohol, and cetanol, is used in combination, the organosiloxane does not hinder solidification of the oily component, and makes it possible to easily prepare a stick-form preparation etc.

Thus, the use of the organosiloxane in the present invention enables a cosmetic which has light touch, good spread, and excellent water repellency, forms uniform cosmetic film, and also successfully achieves favorable feeling on use without strong oily feeling, and which is excellent in stability over time and cosmetic persistence even in a mixture system with an organic ultraviolet absorber, various oil agents, such as silicone, hydrocarbon oil, and ester, or an oily component being solid at 25°C. In addition, it is also possible to easily prepare a stick-form preparation etc.

In the present invention, two or more kinds of the organosiloxane different in viscosity from each other can be used in combination to adjust the feeling on use and volatilization rate. Nevertheless, to achieve slow volatilization rate, only one kind thereof may be used in the cosmetic without requiring precise adjustment.

In the present invention, when the cosmetic is desired to have an ultraviolet-light shielding effect, an ultraviolet absorber is preferably blended.

Since the organosiloxane in the present invention is excellent in compatibility with organic ultraviolet absorbers, the cosmetic containing such an organic ultraviolet absorber has favorable feeling on use, excellent stability over time, and excellent cosmetic persistence.

The ultraviolet absorber is not particularly limited as long as it is a raw material blendable into common cosmetics. Specific examples thereof include homomenthyl salicylate, octocrylene, t-butyl methoxydibenzoylmethane, 4-(2-β-glucopyranosiloxy)propoxy-2-hydroxybenzophenone, octyl salicylate, diethylamino hydroxybenzoyl hexyl benzoate, oxybenzone-6, oxybenzone-9, oxybenzone-1, polysilicone-15, 1-(3,4-dimethoxyphenyl)-4,4-dimethyl-1,3-pentanedione, octyl dimethoxybenzylidene dioxoimidazolidine propionate, oxybenzone-2, terephthalylidene dicamphor sulfonic acid, 2,4,6-tris[4-(2-ethylhexyloxycarbonyl)anilino]-1,3,5-triazine, methylbis(trimethylsiloxy)silyl isopentyl trimethoxycinnamate, drometrizole trisiloxane, 2-ethylhexyl para-dimethylaminobenzoate, isopropyl para-methoxycinnamate, ethylhexyl methoxycinnamate, bis-ethylhexyloxyphenol methoxyphenyl triazine, oxybenzone-3, oxybenzone-4, oxybenzone-5, phenylbenzimidazole sulfonic acid, methylene bis-benzotriazolyl tetramethylbutylphenol, etc. Further, an UVA absorber (e.g., diethylamino hydroxybenzoyl hexyl benzoate, etc.) and an UVB absorber (e.g., ethylhexyl methoxycinnamate, etc.) can be used together, in any combination of UVA absorbers, or in any combination of UVB absorbers.

Among these, particularly preferable are one or more organic ultraviolet absorbers selected from ethylhexyl methoxycinnamate, diethylamino hydroxybenzoyl hexyl benzoate, octyl salicylate, polysilicone-15, t-butyl methoxydibenzoylmethane, oxybenzone, methylene bis-benzotriazolyl tetramethylbutylphenol, bis-ethylhexyloxyphenol methoxyphenyl triazine, and octocrylene. This is because the compatibility with the organosiloxane in the present invention is excellent.

In the present invention, when the cosmetic is desired to be in a solid state, it is preferable to blend a wax, hydrocarbon, ester, higher alcohol, or higher fatty acid which are solid at 25°C.

Even when an oily component solid at 25°C is used in combination, the organosiloxane in the present invention has high affinity for oily components so that a stick-form preparation and so forth can be easily prepared without hindering the solidification of the oily component. Accordingly, the organosiloxane is preferable.

Examples of the oily component being solid at 25°C can include waxes, hydrocarbons, esters, higher alcohols, and higher fatty acids which have a melting point of preferably 40°C or higher, more preferably 60 to 110°C. The oily component is not particularly limited as long as it is a raw material blendable into common cosmetics. Specific examples thereof include vegetable waxes, such as carnauba wax, candelilla wax, rice bran wax, and Japan wax; animal waxes, such as beeswax and spermaceti; hydrocarbon-based waxes, such as solid paraffin, polyethylene, ceresin, ozokerite, and microcrystalline wax; higher alcohols, such as stearyl alcohol, behenyl alcohol, and cetanol; fatty acids, such as stearic acid and behenic acid; and silicone waxes, such as acrylic-silicone resins that are acrylic-silicone graft or block copolymers (acrylic-silicone graft copolymers: KP-561P, 562P, or the like, available from Shin-Etsu Chemical Co., Ltd.) or derivatives thereof. One or more thereamong are preferably selected.

The oily component being solid at 25°C is particularly preferably selected from polyethylene, ceresin, ozokerite, beeswax, microcrystalline wax, stearyl alcohol, behenyl alcohol, and cetanol. This is because the affinity for the organosiloxane in the present invention is particularly high.

The inventive cosmetic can be blended with various optional components used in general cosmetics.

### <Other Optional Components>

As another optional component(s), it is possible to incorporate, for example, (1) an oil agent, (2) a compound having an alcoholic hydroxy group, (3) a surfactant, (4) a powder, (5) a composition including a crosslinked organopolysiloxane and an oil agent in a liquid state at room temperature, (6) a film-forming agent, (7) an antiperspirant, (8) an antimicrobial, and (9) another additive. One kind of these can be used singly, or two or more kinds thereof can be used in appropriate combination.

### (1) Oil Agent

The oil agent may be in a semisolid or liquid state except for the oily component being solid at 25°C described above. Examples of usable oil agent include natural animal and vegetable oil and fat and semisynthetic oil and fat, a hydrocarbon oil, a higher fatty acid, a higher alcohol, and an ester, a silicone oil other than the above-described organosiloxane that is an essential component for exhibiting the effects of the present invention, a fluorine-based oil agent, etc.

### · Natural animal and vegetable oil and fat and Semisynthetic oil and fat

Example of the natural animal and vegetable oil and fat and semisynthetic oil and fat include avocado oil, linseed oil, almond oil, Chinese wax, perilla oil, olive oil, cocoa butter, kapok wax, kaya oil, carnauba wax, cod liver oil, candelilla wax, purified candelilla wax, beef tallow, neat's foot oil, neat's bone fat, hardened beef tallow, apricot kernel oil, spermaceti, hydrogenated oil, wheat germ oil, sesame seed oil, fermented rice extract, rice bran oil, sugar cane wax, sasanqua oil, safflower oil, shea butter, Chinese tung oil, cinnamon oil, jojoba wax, squalane, squalene, shellac wax, turtle oil, soybean oil, tea seed oil, camellia oil, evening primrose oil, corn oil, lard, rapeseed oil, Japanese tung oil, rice bran wax, germ oil, horse fat, persic oil, palm oil, palm kernel oil, castor oil, hydrogenated castor oil, castor oil fatty acid methyl ester, sunflower oil, grape seed oil, bayberry wax, jojoba oil, macadamia nut oil, beeswax, mink oil, meadowfoam oil, cottonseed oil, cotton wax, Japan wax, Japan wax kernel oil, montan wax, palm oil, hydrogenated palm oil, coconut fatty acid triglyceride, mutton tallow, peanut oil, lanolin, liquid lanolin, reduced lanolin, lanolin alcohol, hard lanolin, acetylated lanolin, acetylated lanolin alcohol, lanolin fatty acid isopropyl, polyoxyethylene lanolin alcohol ether, polyoxyethylene lanolin alcohol acetate, lanolin fatty acid polyethylene glycol, polyoxyethylene hydrogenated lanolin alcohol ether, egg yolk oil, etc.

### · Hydrocarbon oil

Examples of the hydrocarbon oil include linear or branched hydrocarbon oils. The hydrocarbon oil may be a volatile hydrocarbon oil or a nonvolatile hydrocarbon oil. Specific examples thereof include α-olefin oligomers, light isoparaffin, isododecane, isohexadecane, light liquid isoparaffin, squalane, synthetic squalane, vegetable squalane, squalene, liquid paraffin, liquid isoparaffin, polyisobutylene, hydrogenated polyisobutene, vaseline, etc.

### . Higher fatty acid

Examples of the higher fatty acid include oleic acid, linoleic acid, linolenic acid, arachidonic acid, eicosapentaenoic acid (EPA), docosahexaenoic acid (DHA), isostearic acid, 12-hydroxystearic acid, etc.

### · Higher alcohol

Examples of the higher alcohol include alcohols having preferably 6 or more carbon atoms. Specific examples of the higher alcohol include oleyl alcohol, isostearyl alcohol, 2-decyltetradecinol, cholesterol, phytosterol, polyoxyethylene cholesterol ether, monostearyl glyceryl ether (batyl alcohol), monooleyl glyceryl ether (selachyl alcohol), etc.

### · Ester

Examples of the ester include diisobutyl adipate, 2-hexyldecyl adipate, di-2-heptylundecyl adipate, N-alkylglycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dioctanoate, neopentyl glycol dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate, cetyl lactate, myristyl lactate, isononyl isononanoate, isotridecyl isononanoate, isopropyl palmitate, 2-ethylhexyl palmitate, 2-hexyldecyl palmitate, 2-heptylundecyl palmitate, cholesteryl 12-hydroxystearate, dipentaerythritol fatty acid ester, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate, isopropyl lauroylsarcosinate, diisostearyl malate, and the like; and glyceride oil, such as acetoglyceryl, glyceryl triisooctanoate, glyceryl triisostearate, glyceryl triisopalmitate, glyceryl tribehenate, glyceryl monostearate, glyceryl di-2-heptylundecanoate, glyceryl trimyristate, and diglyceryl myristate isostearate.

### · Silicone oil

As the silicone oil, it is possible to blend a silicone oil other than the organosiloxane that is an essential component for exhibiting the effects of the present invention. Examples of the silicone oil include linear or branched organopolysiloxanes having low to high viscosity, such as dimethylpolysiloxane (KF-96L-1cs, KF-96L-1.5cs, KF-96L-2cs, and the like; available from Shin-Etsu Chemical Co., Ltd.), octamethyltetrasiloxane (D4), decamethylpentasiloxane (KF-995, available from Shin-Etsu Chemical Co., Ltd.), dodecamethylhexasiloxane (D6), tristrimethylsiloxymethylsilane (TMF-1.5 available from Shin-Etsu Chemical Co., Ltd.), caprylylmethicone, phenyl trimethicone, methylphenyl polysiloxane (KF-54 and KF-54HV, available from Shin-Etsu Chemical Co., Ltd.), diphenylsiloxyphenyl trimethicone (KF-56A available from Shin-Etsu Chemical Co., Ltd.), methylhexyl polysiloxane, methylhydrogenpolysiloxane, and a dimethylsiloxane-methylphenylsiloxane copolymer, amino-modified organopolysiloxane, pyrrolidone-modified organopolysiloxane, pyrrolidone carboxylic acid-modified organopolysiloxane, a silicone rubber such as gum-like dimethylpolysiloxane having a high degree of polymerization, gum-like amino-modified organopolysiloxane, and a gum-like dimethylsiloxane-methylphenylsiloxane copolymer, a cyclic organopolysiloxane solution of silicone gum or rubber, amino acid-modified silicone, fluorine-modified silicone, a dissolved product of silicone including silicone resin, and the like.

### · Fluorine-based oil agent

Examples of the fluorine-based oil agent include perfluoropolyether, perfluorodecalin, perfluorooctane, etc.

### (2) Compound having Alcoholic Hydroxy Group

Examples of the compound having an alcoholic hydroxy group include lower alcohols preferably having 2 to 5 carbon atoms, such as ethanol and isopropanol; and sugar alcohols, such as sorbitol and maltose. Further examples thereof include sterols, such as cholesterol, sitosterol, phytosterol, and lanosterol; etc.

### (3) Surfactant

The surfactant includes nonionic, anionic, cationic, and amphoteric surfactants, but is not particularly limited, and any surfactant may be used as long as it is used for a general cosmetic. Among these surfactants, preferable are a partially crosslinked polyether-modified silicone, a partially crosslinked polyglycerol-modified silicone, a linear or branched polyoxyethylene-modified organopolysiloxane, a linear or branched polyoxyethylene-polyoxypropylene-modified organopolysiloxane, a linear or branched polyoxyethylene/alkyl-co-modified organopolysiloxane, a linear or branched polyoxyethylene-polyoxypropylene/alkyl-co-modified organopolysiloxane, a linear or branched polyglycerol-modified organopolysiloxane, and a linear or branched polyglycerol/alkyl-co-modified organopolysiloxane. In these surfactants, the content of hydrophilic polyoxyethylene group, polyoxyethylene-polyoxypropylene group, or polyglycerol residue preferably accounts for 10 to 70% by mass of the molecule. When the partially crosslinked polyether-modified silicone or the partially crosslinked polyglycerol-modified silicone is used, it is preferable that in a composition including the crosslinked organopolysiloxane and an oil agent in a liquid state at room temperature, the crosslinked organopolysiloxane contain the liquid oil agent in an amount of equal to or more than the weight of the crosslinked organopolysiloxane and be swollen relative to the liquid oil. The liquid oil agent to be used may be the organosiloxane in the present invention, or the oil agent, such as liquid silicone oil, hydrocarbon oil, ester oil, natural animal or vegetable oil, and semisynthetic oil, or fluorine-based oil in the oil agent (1) of the optional component. Examples of the liquid oil agent include a silicone oil having a low viscosity of 0.65 to 100 mm²/s (25°C); hydrocarbon oil, such as liquid paraffin, squalane, isododecane, and isohexadecane; glyceride oil, such as trioctanoin; ester oil, such as isotridecyl isononanoate, N-acyl glutamate, and lauroyl sarcosinate; and natural animal and vegetable oil, such as macadamia nut oil. Specific examples thereof include KSG-210, 240, 310, 320, 330, 340, 320Z, 350Z, 710, 810, 820, 830, 840, 820Z, 850Z, and the like available from Shin-Etsu Chemical Co., Ltd. Specific examples of a surfactant that is not the crosslinked organopolysiloxane include KF-6011, 6013, 6043, 6017, 6028, 6038, 6048, 6100, 6104, 6105, 6106, and the like available from Shin-Etsu Chemical Co., Ltd. In any case, the amount of the surfactant blended is preferably 0.1 to 20% by mass of the whole cosmetic. When the amount is 0.1% or more, functions such as dispersion and emulsification can be sufficiently achieved. When the amount is 20% by mass or less, the cosmetic does not have a sticky feeling of use. Accordingly, the cosmetic is preferable. The HLB of the surfactant is not limited, and is preferably 2 to 14.5 for the purpose of maintaining the water resistance of the cosmetic.

### (4) Powder

Examples of the powder include color pigment, inorganic powder, metal powder, organic powder, inorganic-organic composite powder, etc. Specific examples thereof are as follows.

### · Color pigment

The color pigment is not particularly limited as long as it is a pigment usually used for colorization of cosmetic. It is possible to use any of red iron oxide, yellow iron oxide, white titanium oxide, black iron oxide, red oxide, ultramarine, iron blue, manganese violet, cobalt violet, chromium hydroxide, chromium oxide, cobalt oxide, cobalt titanate, iron oxide-doped titanium oxide, iron titanate, calcined titanium/titanium oxide, lithium cobalt titanate, cobalt titanate, titanium nitride, an inorganic brown pigment such as iron hydroxide and γ-iron oxide, an inorganic yellow pigment such as yellow ocher, a colored pigment such as a laked tar-based dye and a laked natural dye, or the like. Moreover, the pigment may have any shape such as spherical, nearly spherical, rod-like, spindle, petaloid, strip, and amorphous shapes. The geometrical aspect of the pigment is not particularly limited as long as it can impart color to the cosmetic.

### · Inorganic powder

Examples of the inorganic powder include fine particles made of zirconium oxide, zinc oxide, cerium oxide, magnesium oxide, barium sulfate, calcium sulfate, magnesium sulfate, calcium carbonate, magnesium carbonate, talc, cleavable talc, mica, kaolin, sericite, muscovite, synthetic mica, phlogopite, lepidolite, biotite, lithia mica, silicic acid, silicon dioxide, fumed silica, water-containing silicon dioxide, aluminum silicate, magnesium silicate, magnesium aluminum silicate, calcium silicate, barium silicate, strontium silicate, metal tungstate, hydroxyapatite, vermiculite, HIGILITE^{™}, bentonite, montmorillonite, hectorite, zeolite, ceramics, calcium secondary phosphate, alumina, aluminum hydroxide, boron nitride, boron nitride, glass, and the like. Moreover, examples of an inorganic color pearl pigment include pearl pigments, such as titanium oxide-coated mica, bismuth oxychloride, titanium oxide-coated bismuth oxychloride, titanium oxide-coated talc, fish scale guanine, and titanium oxide-coated color mica.

### · Metal powder

Examples of the metal powder include metal fine particles made of aluminum, copper, stainless steel, silver, and the like.

### · Organic powder

Examples of the organic powder include powder made of silicone, polyamide, polyacrylic acid-acrylic acid ester, polyester, polyethylene, polypropylene, polystyrene, styrene-acrylic acid copolymer, divinylbenzene-styrene copolymer, polyurethane, vinyl resin, urea resin, melamine resin, benzoguanamine, polymethylbenzoguanamine, tetrafluoroethylene, polymethyl methacrylate (e.g., polymethyl methacrylate), cellulose, silk, nylon, phenol resin, epoxy resin, polycarbonate, and the like. In particular, examples of the silicone include silicone resin particles (specific examples are KMP-590, 591, 592, and the like, available from Shin-Etsu Chemical Co., Ltd.) and silicone resin-coated silicone rubber powder (specific examples are KSP-100, 101, 102, 105, 300, 411, 441, and the like, available from Shin-Etsu Chemical Co., Ltd.). Examples thereof include metal soap and the like, and specific examples thereof include powder made of zinc stearate, aluminum stearate, calcium stearate, magnesium stearate, zinc myristate, magnesium myristate, zinc cetyl phosphate, calcium cetyl phosphate, sodium zinc cetyl phosphate, and the like. Further, examples of the organic powder include organic dye and the like, and specific examples thereof include tar-based dyes, such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207; and natural dyes, such as carminic acid, laccainic acid, carthamin, brazilin, and crocin.

### · Inorganic-organic composite powder

Examples of the inorganic-organic composite powder include composite powder in which the surface of inorganic powder is coated with organic powder by a publicly-known general method.

Note that as the above-described powders, powder obtained by treating the surface of particles can be used. From the viewpoint of water resistance of the cosmetic, this surface treatment agent is preferably capable of imparting hydrophobicity, and examples of the surface treatment agent for imparting hydrophobicity include, but are not particularly limited to, a silicone treatment agent; a wax; a paraffin; an organic fluorine compound, such as perfluoroalkyl phosphate; a surfactant; an amino acid, such as N-acyl glutamic acid; a metal soap, such as aluminum stearate and magnesium myristate; etc. The silicone treatment agent is more preferable, and examples thereof include silane, such as caprylsilane (AES-3083 available from Shin-Etsu Chemical Co., Ltd.) and trimethoxysilyl dimethicone, or a silylating agent; silicone oil, such as dimethyl silicone (KF-96A series available from Shin-Etsu Chemical Co., Ltd.), methyl hydrogen type polysiloxane (KF-99P, KF-9901, and the like, available from Shin-Etsu Chemical Co., Ltd.), and branched silicone type silicone treatment agent (KF-9908, KF-9909, and the like, available from Shin-Etsu Chemical Co., Ltd.); acrylic-silicone (KP-574 and KP-541, available from Shin-Etsu Chemical Co., Ltd.); etc. Further, these surface-hydrophobizing treatment agents may be used alone or two or more thereof may be used in combination. Specific examples of a surface-treated color pigment include KTP-09 series, and especially KTP-09W, 09R, 09Y, 09B, and the like, available from Shin-Etsu Chemical Co., Ltd.

### (5) Composition including Crosslinked Organopolysiloxane and Oil Agent in liquid state at room temperature

In the composition including a crosslinked organopolysiloxane and an oil agent in a liquid state at room temperature, the crosslinked organopolysiloxane preferably contains the liquid oil in an amount of equal to or more than the weight of the crosslinked organopolysiloxane and swells relative to the liquid oil. The liquid oil agent to be used may be the organosiloxane in the present invention, or the oil agent, such as liquid silicone oil, hydrocarbon oil, ester oil, natural animal or vegetable oil, and semisynthetic oil, or fluorine-based oil in the oil agent (1) of the optional component. Examples thereof include a silicone oil having a low viscosity of 0.65 to 100 mm²/s (25°C), excluding the organosiloxane in the present invention; hydrocarbon oil, such as liquid paraffin, squalane, isododecane, and isohexadecane; glyceride oil, such as trioctanoin; ester oil, such as isotridecyl isononanoate, N-acyl glutamate, and lauroyl sarcosinate; and natural animal or vegetable oil, such as macadamia nut oil. Unlike the component (3) described above, the component (5) is a compound having no polyether or polyglycerol structure in the molecular structure, and specific examples thereof include KSG series (product name), and particularly KSG-15, 16, 016F, 19, 41, 42, 43, 44, 042Z, 045Z, and the like, available from Shin-Etsu Chemical Co., Ltd.

### (6) Film-Forming Agent

The film-forming agent is blended to mainly further maintain persistence of the effect of the cosmetic. The film-forming agent is not particularly limited, and is preferably a silicone-based composition from the viewpoint of imparting water repellency. Specifically, trimethylsiloxysilicate, acrylic-silicone film-forming agent, silicone-modified norbornene, silicone-modified pullulan, or the like can be used. The film-forming agent may be blended in the cosmetic after the film-forming agent is dissolved in an oil agent in a liquid state at room temperature. The liquid oil agent to be used may be the organosiloxane in the present invention, or the oil agent, such as liquid silicone oil, hydrocarbon oil, ester oil, natural animal or vegetable oil, and semisynthetic oil, or fluorine-based oil in the oil agent (1) of the optional component. Examples thereof include a silicone oil having a low viscosity of 0.65 to 100 mm²/s (25°C); hydrocarbon oil, such as liquid paraffin, squalane, isododecane, and isohexadecane; glyceride oil, such as trioctanoin; ester oil, such as isotridecyl isononanoate, N-acyl glutamate, and lauroyl sarcosinate; and natural animal or vegetable oil, such as macadamia nut oil. Specific examples thereof include KF-7312J that is a product of trimethylsiloxysilicate dissolved in silicone, KP-545 and KP-549 that are a product of acrylic-silicone film-forming agent dissolved in silicone, NBN-30-ID that is a product of silicone-modified norbornene dissolved in isododecane, TSPL-30-ID that is a product of silicone-modified pullulan dissolved in isododecane, and TSPL-30-D5 that is a product dissolved in silicone, all of which are available from Shin-Etsu Chemical Co., Ltd., etc.

### (7) Antiperspirant

In a case where the inventive cosmetic is a deodorant, the antiperspirant can be optionally blended. The antiperspirant is not particularly limited as long as it is a component that suppresses generation of sweat by constriction of the skin, and a general-purpose component can be widely used. Examples of the component include chlorohydroxy aluminum, aluminum chloride, aluminum chlorohydroxy allantoinate, aluminum allantoinate, tannic acid, potassium aluminum sulfate, zinc oxide, zinc para-phenolsulfonate, burnt alum, aluminum zirconium tetrachlorohydrate, aluminum zirconium trichlorohydrex glycine, etc. In particular, a component exhibiting a high effect is preferably an adiaphoretic component selected from the group consisting of aluminum halide, aluminum hydroxyhalide, and a complex or mixture thereof with zirconyl oxyhalide and zirconyl hydroxyhalide. It is possible to use the antiperspirant that is blended and dissolved in water or is blended in a powder state into a preparation as it is. As the antiperspirant, a commercially available product can be also used. The commercially available product to be used may be in a form of raw material mixed with another component. The content of the antiperspirant is not particularly limited, and can appropriately vary depending on the amount of the other components to be blended. For the purposes of obtaining a deodorant having excellent adiaphoretic effect as well as obtaining a deodorant having reduced stimulation against the skin, the content thereof preferably falls within a range of 0.001 to 30% by mass, and more preferably 0.01 to 20% by mass, of the whole deodorant.

### (8) Antimicrobial

The antimicrobial is not particularly limited as long as it is a component in which a deodorization effect is obtained by suppressing proliferation of indigenous bacteria that produces a body-odor causing substance on the skin. Examples of generally used antimicrobial drugs include triclosan, benzalkonium chloride, benzethonium chloride, chlorhexidine hydrochloride, chlorhexidine gluconate, halocarban, isomethylphenol, etc. Further, an antimicrobial substance such as an essential oil or extract derived from a crude drug, including a green tea distilled extract and the like, may be blended. As an antimicrobial having a deodorization effect, such as an essential oil or extract derived from a crude drug, it is possible to use, for example, green tea extract, lavender extract, scutellaria root extract, coptis rhizome extract, phellodendron bark extract, Artemisia capillaris extract, Aloe arborescens extract, sophora flavescens root extract, sasa veitchii leaf extract, garlic extract, hamamelis extract, black tea extract, sage leaf extract, zanthoxylum extract, ginger root extract, calamus root extract, English ivy extract, houttuynia cordata extract, peach fruit extract, peach leaf extract, peppermint leaf extract, cnidium rhizome extract, eucalyptus leaf extract, peanut seed coat extract, litchi extract, burnet extract, or the like. One, two or more kinds of vegetable extracts may be mixed.

### (9) Other Additives

Examples of the other additive include an oil-soluble gellant, an ultraviolet absorbing-scattering agent, a moisturizer, a preservative, a fragrance, a salt, an antioxidant, a pH adjuster, a chelator, a refrigerant, an anti-inflammatory agent, a component for skin care (a skin-brightening agent, a cell activator, a rough skin-improving agent, a blood circulation promoter, a skin astringent, an antiseborrheic agent, etc.), vitamin, amino acid, nucleic acid, hormone, an inclusion compound, etc.

### · Oil-soluble gellant

Examples of the oil-soluble gellant include: metal soap, such as aluminum stearate, magnesium stearate, and zinc myristate; amino acid derivative, such as N-lauroyl-L-glutamic acid and α,γ-di-n-butylamine; dextrin fatty acid ester, such as dextrin palmitate, dextrin stearate, and dextrin 2-ethylhexanoate palmitate; sucrose fatty acid ester, such as sucrose palmitate and sucrose stearate; fructooligosaccharide fatty acid ester, such as fructooligosaccharide stearate and fructooligosaccharide 2-ethylhexanoate; benzylidene derivative of sorbitol, such as monobenzylidene sorbitol and dibenzylidene sorbitol; organically modified clay mineral, such as dimethylbenzyldodecylammonium montmorillonite clay, dimethyldioctadecylammonium montmorillonite clay, and dimethyloctadecylammonium hectorite clay; etc.

### · Ultraviolet absorbing-scattering agent

Examples of the ultraviolet absorbing-scattering agent include particles that absorb and scatter ultraviolet light, such as titanium oxide fine particles, iron-containing titanium oxide fine particles, zinc oxide fine particles, cerium oxide fine particles, and composites thereof. A dispersion in which these particles that absorb and scatter ultraviolet light are dispersed in an oil agent in advance can also be used. The oil agent to be used may be the organosiloxane in the present invention, or the oil agent, such as liquid silicone oil, hydrocarbon oil, ester oil, natural animal or vegetable oil, and semisynthetic oil, or fluorine-based oil in the oil agent (1) of the optional component. Examples thereof include a silicone oil having a low viscosity of 0.65 to 100 mm²/s (25°C); hydrocarbon oil, such as liquid paraffin, squalane, isododecane, and isohexadecane; glyceride oil, such as trioctanoin; ester oil, such as isotridecyl isononanoate, N-acyl glutamate, and lauroyl sarcosinate; and natural animal or vegetable oil, such as macadamia nut oil. Specific examples of the dispersion in which particles that absorb and scatter ultraviolet light are dispersed in an oil agent in advance include SPD series (product name), and particularly SPD-T5, Z5, T6, Z6, and the like, available from Shin-Etsu Chemical Co., Ltd.

### · Moisturizer

Examples of the moisturizer include glycerin, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate, polyoxyethylene methyl glucoside, polyoxypropylene methyl glucoside, egg-yolk lecithin, soybean lecithin, phosphatidylcholine, phosphatidylethanolamine, phosphatidylserine, phosphatidylglycerol, phosphatidylinositol, sphingophospholipid, etc.

### · Preservative

Examples of the preservative include alkyl para-hydroxybenzoate, benzoic acid, sodium benzoate, sorbic acid, potassium sorbate, phenoxyethanol, etc. Examples of the antimicrobial include benzoic acid, salicylic acid, phenol, sorbic acid, alkyl para-hydroxybenzoate, para-chloro-meta-cresol, hexachlorophene, trichlorocarbanilide, photosensitizers, phenoxyethanol, etc.

### · Fragrance

The fragrance includes natural fragrance and synthetic fragrance. Examples of the natural fragrance include vegetable fragrance separated from a flower, a leaf, a material, a peel, and the like; and animal fragrance, such as musk and civet. Examples of the synthetic fragrance include hydrocarbon, such as monoterpene; alcohol, such as aliphatic alcohol and aromatic alcohol; aldehyde, such as terpene aldehyde and aromatic aldehyde; ketone, such as alicyclic ketone; ester, such as terpene-based ester; lactone; phenol; oxide; nitrogen-containing compound; acetal; etc.

### · Salt

Examples of the salt include an inorganic salt, an organic acid salt, an amine salt, and an amino acid salt. Examples of the inorganic salt include a sodium salt, a potassium salt, a magnesium salt, a calcium salt, an aluminum salt, a zirconium salt, a zinc salt, and the like of inorganic acid, such as hydrochloric acid, sulfuric acid, carbonic acid, and nitric acid. Examples of the organic acid salt include a salt of organic acid, such as acetic acid, dehydroacetic acid, citric acid, malic acid, succinic acid, ascorbic acid, and stearic acid. Examples of the amine salt and the amino acid salt include a salt of amine, such as triethanolamine; and a salt of amino acid, such as glutamic acid. Besides, it is also possible to use a salt of hyaluronic acid, chondroitin sulfate, or the like; aluminum zirconium glycine complex; an acid-alkali neutralization salt used in preparation of the cosmetic; or the like.

### · Antioxidant

Examples of the antioxidant include, but are not particularly limited to, carotenoid, ascorbic acid and salt thereof, ascorbyl stearate, tocophenol, tocophenol acetate, tocopherol, p-t-butylphenol, butylhydroxyanisol, dibutylhydroxytoluene, phytic acid, ferulic acid, thiotaurine, hypotaurine, sulfite, erythorbic acid and salt thereof, chlorogenic acid, epicatechin, epigallocatechin, epigallocatechin gallate, apigenin, campherol, myricetin, quercetin, etc. One of the antioxidants may be used alone, or two or more thereof may be used in combination.

### • pH adjuster

Examples of the pH adjuster include lactic acid, citric acid, glycolic acid, succinic acid, tartaric acid, dl-malic acid, potassium carbonate, sodium bicarbonate, ammonium bicarbonate, etc.

### · Chelator

Examples of the chelator include alanine, sodium edetate, sodium polyphosphate, sodium metaphosphate, phosphoric acid, etc.

### · Cooling agent

Examples of the cooling agent include L-menthol, camphor, etc.

### · Anti-inflammatory agent

Examples of the anti-inflammatory agent include allantoin, glycyrrhizinic acid and salt thereof, glycyrrhetinic acid and stearyl glycyrrhetinate, tranexamic acid, azulene, etc.

### · Component for skin care

Examples of the component for skin care include: skin-brightening agent, such as placenta extract, arbutin, glutathione, and strawberry geranium extract; cell activator, such as royal jelly, photosensitizer, cholesterol derivative, and calf blood extract; rough skin-improving agent; blood circulation promoter, such as vanillylamide nonylate, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-orizanol; skin astringent, such as zinc oxide and tannic acid; antiseborrheic agent, such as sulfur and thianthrol; etc.

### · Vitamin

Examples of the vitamin include vitamin A, such as vitamin A oil, retinol, retinol acetate, and retinol palmitate; vitamin B, including vitamin B₂, such as riboflavin, riboflavin butyrate, and flavin adenine nucleotide, vitamin B₆, such as pyridoxine hydrochloride, pyridoxine dioctanoate, and pyridoxine tripalmitate, vitamin B₁₂ and derivative thereof, and vitamin B₁₅ and derivative thereof; vitamin C, such as L-ascorbic acid, L-ascorbic acid dipalmitate, sodium L-ascorbic acid-2-sulfate, and dipotassium L-ascorbic acid diphosphate; vitamin D, such as ergocalciferol and cholecalciferol; vitamin E, such as α-tocopherol, β-tocopherol, γ-tocopherol, dl-α-tocopherol acetate, nicotinate, and succinate; nicotinic acid, such as nicotinic acid, benzyl nicotinate, and amide nicotinate; vitamin H; vitamin P; pantothenic acid, such as calcium pantothenate, D-pantothenyl alcohol, pantothenyl ethyl ether, and acetyl pantothenyl ethyl ether; biotin; etc.

### · Amino acid

Examples of the amino acid include glycine, valine, leucine, isoleucine, serine, threonine, phenylalanine, arginine, lysine, aspartic acid, glutamic acid, cystine, cysteine, methionine, tryptophan, etc.

### · Nucleic acid

Examples of the nucleic acid include deoxyribonucleic acid, etc.

### · Hormone

Examples of the hormone include estradiol, ethenyl estradiol, etc.

### · Inclusion compound

Examples of the inclusion compound include cyclodextrin, etc.

Examples of the form of the inventive cosmetic include powder, liquid, solid, etc. The main preparation form includes liquid, cream, aerosol, ointment, emulsified solid, stick, and emulsified stick.

### EXAMPLE

Hereinafter, the present invention will be described more specifically, but the present invention is not limited to these Examples.

An organosiloxane (Production Example 1) used in the inventive cosmetics was produced as follows.

### (Production Example 1) 3,5-Diethyl-1,1,1,3,5,7,7,7-octamethyltetrasiloxane

A 1-L four-necked flask was charged with 282.6 g of 1,1,1,3,5,7,7,7-octamethyltetrasiloxane and 1.4 mg of chloroplatinic acid. An ethylene gas was blown into the mixture under stirring, and a hydrosilylation reaction took place while the temperature was held at 60 to 70°C. The reaction was monitored by gas chromatography. When it was observed that the proportion of a target reached 95% or more, the reaction was terminated. Distillation under reduced pressure (120°C, 10 mmHg) was performed to obtain 3,5-diethyl-1,1,1,3,5,7,7,7-octamethyltetrasiloxane as the target. The boiling point was 227°C and the viscosity was 2.2 mm²/s.

### Solubility Test of Ethylhexyl Methoxycinnamate

The solubility of an organic ultraviolet absorber in the organosiloxane obtained in Production Example 1 and those in other organosiloxanes were evaluated by a method described below. The evaluation results are shown in Table 1.

### (Evaluation Method)

Ethylhexyl methoxycinnamate (sometimes referred to as OMC) and each silicone (sometimes referred to as Sx) weighed at a mass ratio shown in Table 1 were shaken and mixed at room temperature for 24 hours. After 12 days at room temperature, the dissolution state was observed. The results are shown in Table 1.

For each dissolution state, "good" indicates dissolution, and "poor" indicates separation.

**[Table 1]**

| Silicone | Ratio of OMC:Sx | Dissolution state |
|---|---|---|
| KF-96L-1cs | 1 : 1 | poor |
| KF-96L-1.5cs | 1 : 1 | poor |
| KF-96L-2cs | 1 : 1 | poor |
| TMF-1.5 | 1 : 1 | poor |
| D5: KF-995 | 1 : 1 | poor |
| D6 | 1 : 1 | poor |
| Sx of Production Example 1 | 1 : 1 | good |
| Sx of Production Example 1 | 4 : 6 | good |

It was revealed as shown in Table 1 that the organosiloxane of the present invention obtained in Production Example 1 dissolved ethylhexyl methoxycinnamate to higher extent than octamethyltrisiloxane (KF-96L-1cs manufactured by Shin-Etsu Chemical Co., Ltd.), decamethyltetrasiloxane (KF-96L-1.5cs manufactured by Shin-Etsu Chemical Co., Ltd.), undecamethylpentasiloxane (KF-96L-2cs manufactured by Shin-Etsu Chemical Co., Ltd.), tristrimethylsiloxymethylsilane (TMF-1.5 manufactured by Shin-Etsu Chemical Co., Ltd.), decamethylpentasiloxane (KF-995 manufactured by Shin-Etsu Chemical Co., Ltd.), and dodecamethylhexasiloxane (D6).

### Solubility of oily components solid at 25°C and Crystallization Test

The influences of the organosiloxane obtained in Production Example 1 and other organosiloxanes on the solubility and crystallization of solid oily components at 25°C were evaluated by methods described below. Table 2 shows the evaluation results.

### (Evaluation Method)

Oily components being solid at 25°C and silicones weighed at a mass ratio of 1:9 were heated at 105°C for 20 minutes to observe the dissolution state. Then, each heated mixture solution was placed in an aluminum cup, allowed to stand at 25°C for 2 days, and solidified. The penetration score of the solid was measured with a rheometer. The dissolution states according to the visual observation during the heating were shown as follows. "Excellent" indicates uniform dissolution, "good" indicates dispersion, and "poor" indicates separation.

Moreover, in the rheometer measurement, an automatic cone-penetration measurement test apparatus RPM-101 (manufactured by RIGOSHA CO., LTD.) was used, a 1/4 cone was set, and a penetration score after 5 seconds was measured five times. Table 2 shows average penetration scores. A larger value means a softer material.

**[Table 2]**

| Solid oily component | | KF-96L-1cs | KF-96L-2cs | TMF-1.5 | D5 | D6 | Sx of Production Example 1 |
|---|---|---|---|---|---|---|---|
| Polyethylene | dissolution state | good | Poor | poor | good | excellent | excellent |
| | average penetration score | 30.7 | 18.6 | 31.5 | 22.4 | 21.0 | 33.7 |
| Stearyl alcohol | dissolution state | excellent | excellent | excellent | excellent | excellent | excellent |
| | average penetration score | 31.7 | 34.6 | 23.6 | 34.4 | 31.5 | 49.5 |

As shown in Table 2, the organosiloxane of the present invention favorably dissolved all the solid oily components. Particularly, it was revealed that when the polyethylene was used, the solubility was favorable in comparison with that with linear dimethylpolysiloxane. Moreover, it was also found that cooling the heated composition enabled the mixed composition to solidify. It is generally known that when a mixed composition is prepared using an oil agent having high compatibility with such a solid oily component as wax, the composition dissolved by heating and then solidified by cooling has lower hardness. Nevertheless, it was revealed that when the organosiloxane of the present invention was used, the average penetration scores were high for all the oily components, and the solid oily components had high solubility in the organosiloxane.

Hereinafter, the present invention will be specifically described with reference to formulation examples of cosmetics in Examples and Comparative Examples, but the present invention is not limited to these Examples. Unless otherwise specified, a blend mixing amount is represented in terms of % (% by mass).

### (Examples 1, 2, Comparative Examples 1 to 5) W/O type Foundation

W/O type foundations were prepared according to the following production method. The organosiloxane obtained in Production Example 1 was used in Examples 1, 2 but was not used in Comparative Examples 1 to 5. Table 3 shows compositions in Examples 1, 2 and Comparative Examples 1 to 5.

**[Table 3]**

| | | Exanple 1 | Example 2 | Comparative Example 1 | Comparative Example 2 | Comparative Exanple 3 | Comparative Example 4 | Comparative Example 5 |
|---|---|---|---|---|---|---|---|---|
| 1 | Sx of Production Example 1 | 37.5 | 8.0 | - | - | - | - | - |
| 2 | methyl trimethicone (Note 1) | - | - | 37.5 | - | - | - | - |
| 3 | decamethylcyclopentasiloxane (Note 2) | - | 29.5 | - | 37.5 | - | - | 29.5 |
| 4 | dodecamethylcyclohexasiloxane | - | - | - | - | 37.5 | - | - |
| 5 | dimethylsiloxyphenyl trimethicone (Note 3) | - | - | - | - | - | 37.5 | 8.0 |
| 6 | ethylhexyl methoxycinnamate | 7.5 | 7.5 | 7.5 | 7.5 | 7.5 | 7.5 | 7.5 |
| 7 | crosslinked polyether-modified silicone mixture (Note 4) | 3.5 | 3.5 | 3.5 | 3.5 | 3.5 | 3.5 | 3.5 |
| 8 | crosslinked dimethylpolysiloxane mixture (Note 5) | 5.0 | 5.0 | 5.0 | 5.0 | 5.0 | 5.0 | 5.0 |
| 9 | silicone-branched polyether-modified silicone (Note 6) | 2.0 | 2.0 | 2.0 | 2.0 | 2.0 | 2.0 | 2.0 |
| 10 | disteardimonium hectorite | 1.2 | 1.2 | 1.2 | 1.2 | 1.2 | 1.2 | 1.2 |
| 11 | silicone-modified acrylic polymer (Note 7) | 0.3 | 0.3 | 0.3 | 0.3 | 0.3 | 0.3 | 0.3 |
| 12 | silicone-treated titanium oxide (Note 8) | 8.0 | 8.0 | 8.0 | 8.0 | 8.0 | 8.0 | 8.0 |
| 13 | silicone-treated red iron oxide (Note 8) | 1.6 | 1.6 | 1.6 | 1.6 | 1.6 | 1.6 | 1.6 |
| 14 | silicone-treated yellow iron oxide (Note 8) | 0.7 | 0.7 | 0.7 | 0.7 | 0.7 | 0.7 | 0.7 |
| 15 | silicone-treated black iron oxide (Note 8) | 0.2 | 0.2 | 0.2 | 0.2 | 0.2 | 0.2 | 0.2 |
| 16 | dipropylene glycol | 5.0 | 5.0 | 5.0 | 5.0 | 5.0 | 5.0 | 5.0 |
| 17 | methyl para-hydroxybenzoate | 0.3 | 0.3 | 0.3 | 0.3 | 0.3 | 0.3 | 0.3 |
| 18 | sodium citrate | 0.2 | 0.2 | 0.2 | 0.2 | 0.2 | 0.2 | 0.2 |
| 19 | sodium chloride | 0.5 | 0.5 | 0.5 | 0.5 | 0.5 | 0.5 | 0.5 |
| 20 | Ethanol | 3.0 | 3.0 | 3.0 | 3.0 | 3.0 | 3.0 | 3.0 |
| 21 | purified water | 23.5 | 23.5 | 23.5 | 23.5 | 23.5 | 23.5 | 23.5 |
| | Total | 100.0 | 100.0 | 100.0 | 100.0 | 100.0 | 100.0 | 100.0 |

| | | | | | | | | |
|---|---|---|---|---|---|---|---|---|
| (Note 1) manufactured by Shin-Etsu Chemical Co., Ltd.: TMF-1.5 (Note 2) manufactured by Shin-Etsu Chemical Co., Ltd.: KF-995 (Note 3) manufactured by Shin-Etsu Chemical Co., Ltd.: KF-56A (Note 4) manufactured by Shin-Etsu Chemical Co., Ltd.: KSG-210 (mixture of 70-80% dimethylpolysiloxane + 20-30% crosslinked polyether-modified silicone) (Note 5) manufactured by Shin-Etsu Chemical Co., Ltd.: KSG-15 (mixture of 90-96% decamethylcyclopentasiloxane + 4-10% crosslinked dimethylpolysiloxane) (Note 6) manufactured by Shin-Etsu Chemical Co., Ltd.: KF-6028P (Note 7) manufactured by Shin-Etsu Chemical Co., Ltd.: KP-578 (Note 8) manufactured by Shin-Etsu Chemical Co., Ltd.: KTP-09W, R, Y, B (KF-9909-treated color inorganic pigment, W: white, R: red, Y: yellow, B: black) | | | | | | | | |

### (Production Method)

Portions of Components 1 to 6, and Components 7 to 10 were stirred and uniformly mixed. To the mixture, Components 16 to 21 having been prepared separately by uniformly dissolving Components 16 to 20 in Component 21 were quietly added and stirred to obtain an emulsified product. Separately, the remainder of Components 1 to 6, and Components 11 to 15 were treated with a roller, added to the emulsified product, and mixed. The resultant was placed in a predetermined container. In this manner, foundations were obtained.

The use test was performed by ten female experts to evaluate whether the obtained W/O type foundations had (1) good touch (light touch), (2) good spread, (3) good film uniformity, and (4) good cosmetic persistence according to the following evaluation criteria. Moreover, (5) a state after the cosmetic was left to stand at 40°C for one month was also observed. Table 4 shows the evaluation results of Examples 1, 2 and Comparative Examples 1 to 5.

### (Evaluation Criteria)

5 points: very good
4 points: good
3 points: fair
2 points: slightly poor
1 point: poor

The obtained average was judged according to the following criteria.

Judgement of Average:
The obtained average is 4.5 points or more: A
The obtained average is 3.5 points or more and less than 4.5 points: B
The obtained average is 2.5 points or more and less than 3.5 points: C
The obtained average is 1.5 points or more and less than 2.5 points: D
The obtained average is less than 1.5 points: E

**[Table 4]**

| No. | Evaluation item | Example 1 | Example 2 | Comparative Example 1 | Comparative Example 2 | Comparative Example 3 | Comparative Example 4 | Comparative Example 5 |
|---|---|---|---|---|---|---|---|---|
| (1) | Good touch | B | B | B | B | C | D | C |
| (2) | Good spread | A | A | B | A | B | A | A |
| (3) | Good film uniformity | A | A | D | A | D | B | B |
| (4) | Good cosmetic persistence | B | A | C | B | C | C | B |
| (5) | State of cosmetic | stable | stable | separated | separated | separated | stable | thickened |

As apparent from Table 4, it was demonstrated that the foundations of Examples 1, 2 in comparison with Comparative Examples 1 to 5 had light touch, uniform films were obtained, and the foundations had good cosmetic persistence. Moreover, it was revealed that the foundations were excellent in storage stability.

### (Example 3) Sunscreen Emulsion (Shaking)

In accordance with the formulation shown in Table 5, a sunscreen emulsion (shaking) was prepared.

**[Table 5]**

| Component | | Example 3 |
|---|---|---|
| 1 | decamethylcyclopentasiloxane | 22.5 |
| 2 | Sx of Production Example 1 | 9.0 |
| 3 | crosslinked phenyl-modified silicone mixture (Note 1) | 3.0 |
| 4 | silicone-branched alkyl/polyether-co-modified silicone (Note 2) | 2.0 |
| 5 | ethylhexyl methoxycinnamate | 7.5 |
| 6 | hexyl diethylaminohydroxybenzoylbenzoate | 1.0 |
| 7 | octocrylene | 2.5 |
| 8 | disteardimonium hectorite | 1.0 |
| 9 | acrylic-silicone dissolved product (Note 3) | 2.0 |
| 10 | spherical silicone resin composite powder (Note 4) | 0.5 |
| 11 | titanium oxide dispersion (Note 5) | 5.0 |
| 12 | zinc oxide dispersion (Note 6) | 10.0 |
| 13 | 1,3-butylene glycol | 3.0 |
| 14 | sodium citrate | 0.2 |
| 15 | sodium chloride | 0.5 |
| 16 | Ethanol | 5.0 |
| 17 | purified water | 25.3 |
| Total | | 100.0 |

| | | |
|---|---|---|
| [0110] (Note 1) manufactured by Shin-Etsu Chemical Co., Ltd.: KSG-18A (mixture of 80-85% diphenylsiloxy phenyl trimethicone + 15-20% crosslinked phenyl-modified dimethylpolysiloxane) (Note 2) manufactured by Shin-Etsu Chemical Co., Ltd.: KF-6038 (Note 3) manufactured by Shin-Etsu Chemical Co., Ltd.: KP-545 (dissolved product of 70% decamethylcyclopentasiloxane + 30% acrylic silicone-based graft copolymer) (Note 4) manufactured by Shin-Etsu Chemical Co., Ltd.: KSP-105 (composite powder of silicone rubber coated with silicone resin and having an average particle diameter of 2 um) (Note 5) manufactured by Shin-Etsu Chemical Co., Ltd.: SPD-T5 (dispersion of 40% titanium oxide fine particles in decamethylcyclopentasiloxane) (Note 6) manufactured by Shin-Etsu Chemical Co., Ltd.: SPD-Z5 (dispersion of 60% zinc oxide fine particles in decamethylcyclopentasiloxane) | | |

### (Production Method)

A: Components 1 to 9 were uniformly mixed.
B: Component 10 was added to A and uniformly dispersed.
C: Components 13 to 16 were added to Component 17 and dissolved.
D: C was gradually added to B and emulsified, and Components 11, 12 were added to obtain a sunscreen emulsion.

It was found that the sunscreen emulsion obtained as described above had light spreadability, exhibited dry touch but no stickiness, and did not change due to temperature or over time, and both the usability and stability were quite excellent.

### (Example 4) Sunscreen Cream

In accordance with the formulation shown in Table 6, a sunscreen cream was prepared.

**[Table 6]**

| Component | | Example 4 |
|---|---|---|
| 1 | Sx of Production Example 1 | 30.0 |
| 2 | Squalene | 3.0 |
| 3 | silicone-branched alkyl/polyglycerol-co-modified silicone (Note 1) | 4.0 |
| 4 | ethylhexyl methoxycinnamate | 7.5 |
| 5 | t-butylmethoxydibenzoylmethane | 3.0 |
| 6 | polysilicone-15 | 1.0 |
| 7 | distearyldimethylammonium chloride | 1.0 |
| 8 | vitamin E acetate | 0.1 |
| 9 | ethanol | 1.0 |
| 10 | sodium citrate | 0.5 |
| 11 | magnesium sulfate | 0.5 |
| 12 | preservative | 0.3 |
| 13 | purified water | 48.1 |
| Total | | 100.0 |

| | | |
|---|---|---|
| [0115] (Note 1) manufactured by Shin-Etsu Chemical Co., Ltd.: KF-6105 | | |

### (Production Method)

A: Components 1 to 8 were uniformly mixed.
B: Component 9 to 12 were uniformly dissolved in Component 13.
C: B was gradually added to A under stirring, and emulsified to obtain a sunscreen cream.

It was found that the sunscreen cream obtained as described above had fine texture and light spreadability, gave wet and fresh feeling, did not attach sand at all due to absence of stickiness, and had quite favorable usability. Further, it was found that the sunscreen cream did not change due to temperature or over time and had excellent stability.

### (Example 5) Sunscreen Cream

In accordance with the formulation shown in Table 7, a sunscreen cream was prepared.

**[Table 7]**

| Component | | Example 5 |
|---|---|---|
| 1 | Sx of Production Example 1 | 16.0 |
| 2 | crosslinked alkyl/polyether-co-modified silicone mixture (Note 1) | 3.5 |
| 3 | crosslinked phenyl-modified silicone mixture (Note 2) | 3.0 |
| 4 | alkyl/polyether-co-modified silicone (Note 3) | 1.5 |
| 5 | trimethylsiloxy silicate dissolved product (Note 4) | 3.0 |
| 6 | ethylhexyl methoxycinnamate | 7.5 |
| 7 | t-butylmethoxydibenzoylmethane | 3.0 |
| 8 | ethylhexyl salicylate | 3.0 |
| 9 | 1,3-butylene glycol | 5.0 |
| 10 | sodium citrate | 0.5 |
| 11 | sodium chloride | 1.0 |
| 12 | preservative | 0.3 |
| 13 | purified water | 52.7 |
| Total | | 100.0 |

| | | |
|---|---|---|
| [0120] (Note 1) manufactured by Shin-Etsu Chemical Co., Ltd.: KSG-310 (mixture of 65-75% mineral oil + 25-35% alkyl branched, crosslinked polyether-modified silicone) (Note 2) manufactured by Shin-Etsu Chemical Co., Ltd.: KSG-18A (mixture of 80-85% diphenylsiloxy phenyl trimethicone + 15-20% crosslinked phenyl-modified dimethylpolysiloxane) (Note 3) manufactured by Shin-Etsu Chemical Co., Ltd.: KF-6048 (Note 4) manufactured by Shin-Etsu Chemical Co., Ltd.: KF-7312J (dissolved product of 50% decamethylcyclopentasiloxane + 50% silicone resin) | | |

### (Production Method)

A: Components 1 to 8 were uniformly mixed.
B: Components 9 to 12 were uniformly dissolved in Component 13.
C: B was gradually added to A under stirring, and emulsified to obtain a sunscreen cream.

The sunscreen cream obtained as described above had light spreadability, exhibited fresh feeling and no stickiness, and had favorable cosmetic persistence, and thus the anti-UV effect was also sustained. Moreover, it was found that the sunscreen cream did not change due to temperature or over time, and both the usability and stability were quite excellent.

### (Example 6) Nonaqueous Mousse Foundation

In accordance with the formulation shown in Table 8, a nonaqueous mousse foundation was prepared.

**[Table 8]**

| Component | | Example 6 |
|---|---|---|
| 1 | crosslinked polyether-modified silicone mixture (Note 1) | 18.0 |
| 2 | dimethylpolysiloxane 6 mm²/s | 1.0 |
| 3 | Sx of Production Example 1 | 11.0 |
| 4 | ethylhexyl methoxycinnamate | 5.0 |
| 5 | jojoba oil | 1.0 |
| 6 | silylated silicic anhydride (Note 2) | 0.8 |
| 7 | silicone-treated red iron oxide (Note 3) | 0.2 |
| 8 | silicone-treated yellow iron oxide (Note 3) | 1.0 |
| 9 | silicone-treated black iron oxide (Note 3) | 0.02 |
| 10 | silicone-treated titanium oxide (Note 3) | 5.0 |
| 11 | silicone-treated talc (Note 4) | 11.6 |
| 12 | trimethylsiloxy silicate dissolved product (Note 5) | 4.0 |
| 13 | decamethylcyclopentasiloxane | 25.18 |
| 14 | spherical silicone composite powder (Note 6) | 6.0 |
| 15 | spherical polymethylsilsesquioxane powder (Note 7) | 3.0 |
| 16 | spherical alkyl polymethacrylate | 7.0 |
| 17 | antioxidant | 0.2 |
| Total | | 100.0 |

| | | |
|---|---|---|
| (Note 1) manufactured by Shin-Etsu Chemical Co., Ltd.: KSG-240 (mixture of 75-85% decamethylcyclopentasiloxane + 15-25% crosslinked polyether-modified silicone) (Note 2) surface-hydrophobized fumed silica manufactured by Nippon Aerosil Co., Ltd.: AEROSIL R-972 (Note 3) manufactured by Shin-Etsu Chemical Co., Ltd.: KTP-09W, R, Y, B (KF-9909-treated color inorganic pigment, W: white, R: red, Y: yellow, B: black) (Note 4) manufactured by Shin-Etsu Chemical Co., Ltd.: treated with KF-9909 (Note 5) manufactured by Shin-Etsu Chemical Co., Ltd.: KF-7312J (dissolved product of 50% decamethylcyclopentasiloxane + 50% silicone resin) (Note 6) manufactured by Shin-Etsu Chemical Co., Ltd.: KSP-411 (composite powder of silicone rubber coated with silicone resin and having an average particle diameter of 12 um) (Note 7) manufactured by Shin-Etsu Chemical Co., Ltd.: KMP-590 | | |

### (Production Method)

Components 1 to 10 were uniformly mixed by a roller treatment. To this mixture, Components 11 to 17 were added and uniformly mixed to obtain a nonaqueous mousse foundation.

It was verified that the foundation obtained as described above had an appearance firmly hardened like a mousse, had light spreadability, excellent feeling on use with no stickiness or oily feeling, and quite favorable cosmetic persistence. Moreover, the foundation had no oil exudation or the like despite temperature or time and was excellent in stability.

### (Example 7) Nonaqueous Eye Wrinkle Cream

In accordance with the formulation shown in Table 9, a nonaqueous eye wrinkle cream was prepared.

**[Table 9]**

| Component | | Example 7 |
|---|---|---|
| 1 | crosslinked polyether-modified silicone mixture (Note 1) | 5.0 |
| 2 | crosslinked alkyl-modified silicone mixture (Note 2) | 7.0 |
| 3 | crosslinked dimethylpolysiloxane mixture (Note 3) | 55.0 |
| 4 | decamethylcyclopentasiloxane | 8.0 |
| 5 | Sx of Production Example 1 | 6.0 |
| 6 | jojoba oil | 2.0 |
| 7 | spherical silicone composite powder (Note 4) | 12.0 |
| 8 | highly polymerized dimethylpolysiloxane dissolved product (Note 5) | 5.0 |
| Total | | 100.0 |

| | | |
|---|---|---|
| (Note 1) manufactured by Shin-Etsu Chemical Co., Ltd.: KSG-210 (mixture of 70-80% dimethylpolysiloxane + 20-30% crosslinked polyether-modified silicone) (Note 2) manufactured by Shin-Etsu Chemical Co., Ltd.: KSG-41A (mixture of 70-80% mineral oil + 20-30% alkyl branched, crosslinked dimethylpolysiloxane) (Note 3) manufactured by Shin-Etsu Chemical Co., Ltd.: KSG-15 (mixture of 90-96% decamethylcyclopentasiloxane + 4-10% crosslinked dimethylpolysiloxane) (Note 4) manufactured by Shin-Etsu Chemical Co., Ltd.: KSP-101 (composite powder of silicone rubber coated with silicone resin and having an average particle diameter of 12 µm) (Note 5) manufactured by Shin-Etsu Chemical Co., Ltd.: KF-9028 (dissolved product of 80% decamethylcyclopentasiloxane + 20% silicone rubber) | | |

### (Production Method)

A: Components 1 to 8 were uniformly mixed to obtain an eye wrinkle cream.

It was found that the eye wrinkle cream obtained as described above had light spreadability, gave feeling on use with no stickiness or oily feeling but wet feeling and good compatibility with the skin, did not change due to temperature or over time, and had excellent stability.

### (Example 8) Rinse-Off Pack Cosmetic

In accordance with the formulation shown in Table 10, a rinse-off pack cosmetic was prepared.

**[Table 10]**

| Component | | Example 8 |
|---|---|---|
| 1 | dimethylpolysiloxane (6cs) | 3.0 |
| 2 | Sx of Production Example 1 | 3.0 |
| 3 | silicone-branched polyglycerol-modified silicone (Note 1) | 2.0 |
| 4 | kaolin | 30.0 |
| 5 | carboxyvinyl polymer | 0.4 |
| 6 | 1,3-BG | 10.0 |
| 7 | glycerol | 20.0 |
| 8 | preservative | 0.1 |
| 9 | fragrance | 0.1 |
| 10 | purified water | 31.4 |
| Total | | 100.0 |

| | | |
|---|---|---|
| (Note 1) manufactured by Shin-Etsu Chemical Co., Ltd.: KF-6100 | | |

### (Production Method)

A: Components 1 to 3 and 8 were mixed.
B: Components 5 to 7 and 10 were uniformly mixed, then mixed with Components 4, 9, and stirred.
C: A was added to B and emulsified to obtain a paste-state rinse-off pack cosmetic.

It was found that the rinse-off pack cosmetic obtained as described above had light spreadability during the application, had excellent cleaning effect and quite excellent feeling on use, such as imparting wet feeling with no stickiness and smooth touch to the skin after the rinsing off, and had excellent stability.

### (Example 9) Eye Shadow

In accordance with the formulation shown in Table 11, an eye shadow was prepared.

**[Table 11]**

| Component | | Example 9 |
|---|---|---|
| 1 | isododecane | 25.5 |
| 2 | acrylic-silicone dissolved product (Note 1) | 20.0 |
| 3 | long-chain alkyl-containing acrylic-silicone resin (Note 2) | 2.0 |
| 4 | alkyl-modified spherical silicone composite powder (Note 3) | 6.0 |
| 5 | methyl trimethicone (Note 4) | 3.0 |
| 6 | Sx of Production Example 1 | 5.0 |
| 7 | vaseline | 5.0 |
| 8 | crosslinked alkyl/polyether-co-modified silicone mixture (Note 5) | 5.0 |
| 9 | amorphous silicic anhydride (Note 6) | 1.0 |
| 10 | barium sulfate | 5.0 |
| 11 | organic pigment | 0.2 |
| 12 | alkyl/silicone branched silicone-treated yellow iron oxide (Note 7) | 1.0 |
| 13 | alkyl/silicone branched silicone-treated titanium oxide (Note 7) | 1.0 |
| 14 | alkyl/silicone branched silicone-treated titanium mica (Note 8) | 20.0 |
| 15 | tocopherol | 0.2 |
| 16 | fragrance | 0.1 |
| Total | | 100.0 |

| | | |
|---|---|---|
| (Note 1) manufactured by Shin-Etsu Chemical Co., Ltd.: KP-550 (dissolved product of 60% isododecane + 40% acrylic silicone-based graft copolymer) (Note 2) manufactured by Shin-Etsu Chemical Co., Ltd.: KP-561P (silicone wax having a melting point of 30°C) (Note 3) manufactured by Shin-Etsu Chemical Co., Ltd.: KSP-441 (composite powder of alkyl-modified silicone rubber coated with silicone resin and having an average particle diameter of 12 um) (Note 4) manufactured by Shin-Etsu Chemical Co., Ltd.: TMF-1.5 (Note 5) manufactured by Shin-Etsu Chemical Co., Ltd.: KSG-320 (mixture of 70-80% isododecane + 20-30% alkyl branched, crosslinked polyether-modified silicone) (Note 6) surface-hydrophobized fumed silica manufactured by Nippon Aerosil Co., Ltd.: AEROSIL R-972 (Note 7) manufactured by Shin-Etsu Chemical Co., Ltd.: KTP-09W, Y (KF-9909-treated color inorganic pigment, W: white, Y: yellow) (Note 8) manufactured by Shin-Etsu Chemical Co., Ltd.: treated with KF-9909 | | |

### (Production Method)

A: Components 1 to 9 were mixed and uniformly dispersed.
B: Components 10 to 16 were added to the mixture obtained in A and uniformly dispersed to obtain an eye shadow.

The eye shadow obtained as described above had good removability, light spreadability, and feeling on use with no oily or powdery feeling. It was also verified that the eye shadow had favorable water resistance, water repellency, perspiration resistance, and persistence, was unlikely to cause the make-up to smear, did not change despite temperature change and over time, and had excellent stability.

### (Example 10) Eye Liner

In accordance with the formulation shown in Table 12, an eye liner was prepared.

**[Table 12]**

| Component | | Example 10 |
|---|---|---|
| 1 | Sx of Production Example 1 | 22.0 |
| 2 | dimethylpolysiloxane (6cs) | 5.0 |
| 3 | crosslinked alkyl-modified silicone mixture (Note 1) | 5.0 |
| 4 | jojoba oil | 2.0 |
| 5 | silicone-branched alkyl/polyether-co-modified silicone (Note 2) | 3.0 |
| 6 | silicone-treated black iron oxide (Note 3) | 20.0 |
| 7 | ethanol | 5.0 |
| 8 | preservative | 0.1 |
| 9 | purified water | 37.9 |
| Total | | 100.0 |

| | | |
|---|---|---|
| (Note 1) manufactured by Shin-Etsu Chemical Co., Ltd.: KSG-42A (mixture of 75-85% isododecane + 15-25% alkyl branched, crosslinked dimethylpolysiloxane) (Note 2) manufactured by Shin-Etsu Chemical Co., Ltd.: KF-6038 (Note 3) manufactured by Shin-Etsu Chemical Co., Ltd.: treated with KF-9901 | | |

### (Production Method)

A: Components 1 to 5 were heated and mixed, and Component 6 was added thereto and uniformly dispersed.
B: Components 7 to 9 were heated and dissolved.
C: B was gradually added to A under stirring, and emulsified to obtain an eye liner.

It was found that the eye liner obtained as described above spread lightly, gave feeling on use with no oily feeling or powdery feeling but wetting and refresh feelings, had favorable water resistance, water repellency, perspiration resistance, and persistence, was unlikely to cause the make-up to smear, did not change due to temperature or over time, and had excellent stability.

### (Example 11) W/O Cleansing Cream

In accordance with the formulation shown in Table 13, a W/O cleansing cream was prepared.

**[Table 13]**

| Component | | Example 11 |
|---|---|---|
| 1 | dimethylpolysiloxane (6cs) | 10.0 |
| 2 | methylphenylpolysiloxane | 15.0 |
| 3 | liquid paraffin | 8.0 |
| 4 | isostearic acid | 1.0 |
| 5 | Sx of Production Example 1 | 11.0 |
| 6 | dextrin fatty acid ester | 0.8 |
| 7 | polyether-modified silicone (Note 1) | 4.0 |
| 8 | glycerol | 10.0 |
| 9 | sodium citrate | 0.2 |
| 10 | sodium chloride | 1.0 |
| 11 | preservative | 0.1 |
| 12 | fragrance | 0.1 |
| 13 | purified water | 38.8 |
| Total | | 100.0 |

| | | |
|---|---|---|
| (Note 1) manufactured by Shin-Etsu Chemical Co., Ltd.: KF-6017 | | |

A: Components 1 to 7 were heated and mixed.
B: Components 8 to 11 and 13 were heated and dissolved.
C: B was gradually added to A under stirring, emulsified, and cooled, and Component 12 was added thereto to obtain a cleansing cream.

It was found that the cleansing cream obtained as described above had fine texture and light spreadability, gave wet and fresh feeling with no stickiness or oily feeling, gave refreshing feeling on use, had high cleansing effect, did not change due to temperature or over time, and had excellent stability.

### (Example 12) Lip Cream

In accordance with the formulation shown in Table 14, a lip cream was prepared.

**[Table 14]**

| Component | | Example 12 |
|---|---|---|
| 1 | palmitic acid/dextrin ethylhexanoate (Note 1) | 9.0 |
| 2 | Sx of Production Example 1 | 7.0 |
| 3 | acrylic-silicone dissolved product (Note 2) | 5.0 |
| 4 | crosslinked alkyl-modified silicone mixture (Note 3) | 8.0 |
| 5 | silicone-branched alkyl/polyglycerol-co-modified silicone (Note 4) | 2.0 |
| 6 | decamethylcyclopentasiloxane | 46.0 |
| 7 | 1,3-butylene glycol | 5.0 |
| 8 | purified water | 10.8 |
| 9 | colorant | 1.2 |
| 10 | titanium oxide-coated mica | 6.0 |
| Total | | 100.0 |

| | | |
|---|---|---|
| (Note 1) manufactured by Chiba Flour Milling Co., Ltd.: Rheopearl TT (Note 2) manufactured by Shin-Etsu Chemical Co., Ltd.: KP-545 (dissolved product of 70% decamethylcyclopentasiloxane + 30% acrylic silicone-based graft copolymer) (Note 3) manufactured by Shin-Etsu Chemical Co., Ltd.: KSG-43 (mixture of 65-75% triethylhexanoin + 25-35% alkyl branched, crosslinked dimethylpolysiloxane) (Note 4) manufactured by Shin-Etsu Chemical Co., Ltd.: KF-6105 | | |

### (Production Method)

A: After Component 9 was mixed with a portion of Component 2 and dispersed using a roller mill, the obtained dispersion, Component 1, the remainder of Component 2, and Components 3 to 6 were heated and mixed.
B: Components 7, 8 were heated, added to the mixture obtained in A, emulsified, and then cooled.
C: Component 10 was added to the emulsified product obtained in B to obtain a lip cream.

The obtained lip cream spread lightly, had no stickiness or oily feeling, and formed a durable film on the lip.

### (Example 13) Mascara

In accordance with the formulation shown in Table 15, a mascara was prepared.

**[Table 15]**

| Component | | Example 13 |
|---|---|---|
| 1 | silicone-branched polyether-modified silicone (Note 1) | 1.0 |
| 2 | dimethyldistearylammonium hectorite | 4.0 |
| 3 | isododecane | 39.5 |
| 4 | acrylic-silicone dissolved product (Note 2) | 20.0 |
| 5 | palmitic acid/dextrin ethylhexanoate (Note 3) | 3.0 |
| 6 | ceresin | 2.5 |
| 7 | long-chain alkyl-containing acrylic-silicone resin (Note 4) | 2.0 |
| 8 | beeswax | 2.5 |
| 9 | Sx of Production Example 1 | 5.0 |
| 10 | hydrogenated lecithin | 0.5 |
| 11 | silica | 3.0 |
| 12 | talc | 12.0 |
| 13 | hydrophobized colorant (Note 5) | 5.0 |
| Total | | 100.0 |

| | | |
|---|---|---|
| (Note 1) manufactured by Shin-Etsu Chemical Co., Ltd.: KF-6028P (Note 2) manufactured by Shin-Etsu Chemical Co., Ltd.: KP-550 (dissolved product of 60% isododecane + 40% acrylic silicone-based graft copolymer) (Note 3) manufactured by Chiba Flour Milling Co., Ltd.: Rheopearl TT (Note 4) manufactured by Shin-Etsu Chemical Co., Ltd.: KP-562P (silicone wax having a melting point of 50°C) (Note 5) manufactured by Shin-Etsu Chemical Co., Ltd.: KTP-09B (KF-9909-treated color inorganic pigment, B: black) | | |

### (Production Method)

A: Components 1 to 3 were uniformly mixed.
B: Components 4 to 10 were heated, stirred, and dissolved, and the mixture obtained in A and pulverized Components 11, 12, 13 were added thereto, uniformly mixed, and then cooled.

The obtained mascara was not sticky, spread lightly, was easily applied to the eyelash, and had quite favorable cosmetic persistence.

### (Example 14) Cleansing Oil

In accordance with the formulation shown in Table 16, a cleansing oil was prepared.

**[Table 16]**

| Component | | Example 14 |
|---|---|---|
| 1 | mineral oil | 30.0 |
| 2 | isopropyl myristate | 2.0 |
| 3 | Sx of Production Example 1 | 54.9 |
| 4 | PEG-6 diisostearate | 1.0 |
| 5 | tocopherol acetate | 0.1 |
| 6 | PEG-20 glyceryl triisostearate | 10.0 |
| 7 | glycerol | 1.0 |
| 8 | purified water | 1.0 |
| Total | | 100.0 |

### (Production Method)

A: Components 1 to 6 were uniformly mixed.
B: Components 7, 8 were stirred, dissolved, added to the mixture obtained in A, and uniformly mixed to obtain a cleansing oil.

The obtained cleansing oil was not sticky, formed a uniform oily film, easily spread, and had quite high cleaning effect.

### (Example 15) Deodorant Stick

In accordance with the formulation shown in Table 17, a deodorant stick was prepared.

**[Table 17]**

| Component | | Example 15 |
|---|---|---|
| 1 | chlorohydroxy aluminum | 23.0 |
| 2 | Sx of Production Example 1 | 36.5 |
| 3 | stearyl alcohol | 8.0 |
| 4 | talc | 14.88 |
| 5 | fragrance | 0.1 |
| 6 | BHT | 0.02 |
| 7 | paraffin (solid) | 2.0 |
| 8 | mineral oil | 14.5 |
| 9 | alkyl/polyether-co-modified silicone (Note 1) | 1.0 |
| Total | | 100.0 |

| | | |
|---|---|---|
| (Note 1) manufactured by Shin-Etsu Chemical Co., Ltd.: KF-6048 | | |

### (Production Method)

A: Components 2, 3, 6 to 9 were heated, dissolved, and mixed.
B: Components 1, 4 were uniformly mixed with A, and uniformly dispersed and mixed using a mixer.
C: Component 5 was added to the resultant and uniformly mixed.
D: The mixture was poured into a mold, cooled, and solidified.

The feeling on use of the deodorant stick of Example 15 was evaluated. Table 18 shows the result.

### Usability Evaluation Method

Ten experts actually used the deodorant stick to evaluate the feeling on use.

### Evaluation Criteria

The judgement was performed according to the following criteria.
excellent: five or more experts evaluated it as favorable.
good: three or more experts evaluated it as favorable.
poor: less than three experts evaluated it as favorable.

**[Table 18]**

| | Example 15 |
|---|---|
| Dry feeling | excellent |
| Sticky feeling | excellent |
| Deodorization effect | excellent |

It can be seen that Example 15, the deodorant of the present invention, had no excessive dry feeling or sticky feeling, and was excellent in persistence of deodorization effect.

### (Example 16) Deodorant Spray

In accordance with the formulation shown in Table 19, a deodorant spray was prepared.

**[Table 19]**

| Component | | Example 16 |
|---|---|---|
| 1 | chlorohydroxy aluminum | 30.0 |
| 2 | silicic anhydride | 15.0 |
| 3 | Sx of Production Example 1 | 10.0 |
| 4 | silicone-treated talc (Note 1) | 14.88 |
| 5 | fragrance | 0.1 |
| 6 | BHT (dibutylhydroxytoluene) | 0.02 |
| 7 | zinc oxide | 5.0 |
| 8 | triclosan | 0.1 |
| 9 | isopropyl myristate | 21.9 |
| 10 | silicone-branched alkyl/polyglycerol-co-modified silicone (Note 2) | 3.0 |
| Total | | 100.0 |

| | | |
|---|---|---|
| (Note 1) manufactured by Shin-Etsu Chemical Co., Ltd.: treated with KF-9909 (Note 2) manufactured by Shin-Etsu Chemical Co., Ltd.: KF-6105 | | |

### (Production Method)

A: Components 2 to 4, 6 to 10 were uniformly mixed.
B: Component 1 was uniformly mixed with A, and uniformly dispersed and mixed using a mixer.
C: Component 5 was added to the resultant and uniformly mixed.
D: A spray container was filled with 10 parts of the raw liquid Component C and 90 parts of LPG.

The feeling on use of the deodorant spray of Example 16 was evaluated. Table 20 shows the result.

### Usability Evaluation Method

Ten experts actually used the deodorant spray to evaluate the feeling on use.

### Evaluation Criteria

The judgement was performed according to the following criteria.
excellent: five or more experts evaluated it as favorable.
good: three or more experts evaluated it as favorable.
poor: less than three experts evaluated it as favorable.

**[Table 20]**

| | Example 16 |
|---|---|
| Dry feeling | excellent |
| Sticky feeling | excellent |
| Deodorization effect | excellent |

It can be seen that Example 16, the deodorant of the present invention, had no excessive dry feeling or sticky feeling, and was excellent in persistence of deodorization effect.

### (Example 17) Nonaqueous Deodorant Cream

In accordance with the formulation shown in Table 21, a nonaqueous deodorant cream was prepared.

**[Table 21]**

| Component | | Example 17 |
|---|---|---|
| 1 | aluminum zirconium trichlorohydrex glycine | 19.0 |
| 2 | Sx of Production Example 1 | 30.0 |
| 3 | crosslinked dimethylpolysiloxane mixture (Note 1) | 21.5 |
| 4 | spherical silicone composite powder (Note 2) | 10.0 |
| 5 | neopentyl glycol dioctanoate | 9.68 |
| 6 | fragrance | 0.1 |
| 7 | BHT (dibutylhydroxytoluene) | 0.02 |
| 8 | citric acid | 0.1 |
| 9 | benzyl alcohol | 0.1 |
| 10 | silica dimethyl silylate | 0.5 |
| 11 | polyethylene | 3.0 |
| 12 | ceresin | 6.0 |
| Total | | 100.0 |

| | | |
|---|---|---|
| (Note 1) manufactured by Shin-Etsu Chemical Co., Ltd.: KSG-15 (mixture of 90-96% decamethylcyclopentasiloxane + 4-10% crosslinked dimethylpolysiloxane) (Note 2) manufactured by Shin-Etsu Chemical Co., Ltd.: KSP-100 (composite powder of silicone rubber coated with silicone resin and having an average particle diameter of 5 um) | | |

### (Production Method)

A: Components 2, 3, 5, 7, 9, 11, 12 were heated and uniformly mixed.
B: Component 10 was uniformly mixed with A, and uniformly dispersed and mixed using a mixer.
C: Components 1, 4, 8 were added to B, and uniformly mixed.
D: Component 6 was added to C, uniformly mixed, and then placed in a container.

Example 17 was a nonaqueous deodorant cream which was very smoothly applied and spread, had good spread but no excessive dry feeling or sticky feeling, and was excellent in persistence of deodorization effect.

### (Example 18) W/O Deodorant Cream

In accordance with the formulation shown in Table 22, a W/O deodorant cream was prepared.

**[Table 22]**

| Component | | Example 18 |
|---|---|---|
| 1 | crosslinked polyether-modified silicone mixture (Note 1) | 3.0 |
| 2 | crosslinked dimethylpolysiloxane mixture | 2.0 |
| 3 | silicone-branched polyether-modified silicone (Note 3) | 2.0 |
| 4 | ethylhexyl palmitate | 5.0 |
| 5 | isopropyl methylphenol | 0.1 |
| 6 | chlorohydroxy aluminum | 5.0 |
| 7 | benzalkonium chloride | 0.1 |
| 8 | Sx of Production Example 1 | 15.0 |
| 9 | glycerol | 5.0 |
| 10 | 1,3-BG | 5.0 |
| 11 | ethanol | 5.0 |
| 12 | phenoxyethanol | 0.3 |
| 13 | purified water | 52.5 |
| Total | | 100.0 |

| | | |
|---|---|---|
| (Note 1) manufactured by Shin-Etsu Chemical Co., Ltd.: KSG-210 (mixture of 70-80% dimethylpolysiloxane + 20-30% crosslinked polyether-modified silicone) (Note 2) manufactured by Shin-Etsu Chemical Co., Ltd.: KSG-15 (mixture of 90-96% decamethylcyclopentasiloxane + 4-10% crosslinked dimethylpolysiloxane) (Note 3) manufactured by Shin-Etsu Chemical Co., Ltd.: KF-6028P | | |

### (Production Method)

A: Components 1 to 4 and 8 were uniformly mixed.
B: Components 5 to 7 were uniformly mixed with A, and uniformly dispersed and mixed using a mixer.
C: Components 9 to 13 were dissolved.
D: C was added to B, emulsified, and then placed in a container.

Example 18 was a W/O deodorant cream which had fresh feeling on use, was very smoothly applied and spread, had good spread and no excessive dry feeling or sticky feeling during the use, and was excellent in persistence of deodorization effect.

### (Example 19) Hair Oil

In accordance with the formulation shown in Table 23, a hair oil was prepared.

**[Table 23]**

| Component | | Example 19 |
|---|---|---|
| 1 | Sx of Production Example 1 | 68.0 |
| 2 | hydrogenated polyisobutene | 10.3 |
| 3 | trimethylsiloxy silicate dissolved product (Note 1) | 3.0 |
| 4 | dimethiconol | 7.0 |
| 5 | diethylhexyl succinate | 10.0 |
| 6 | highly polymerized dimethylpolysiloxane dissolved product (Note 2) | 1.5 |
| 7 | tocopherol | 0.1 |
| 8 | fragrance | 0.1 |
| Total | | 100.0 |

| | | |
|---|---|---|
| (Note 1) manufactured by Shin-Etsu Chemical Co., Ltd.: KF-7312J (dissolved product of 50% decamethylcyclopentasiloxane + 50% silicone resin) (Note 2) manufactured by Shin-Etsu Chemical Co., Ltd.: KF-9030 | | |

### (Production Method)

A: Components 1 to 8 were uniformly mixed to obtain a hair oil.

Example 19 was a hair oil which had light spreadability and gave the hair glossiness and smoothness.

It should be noted that the present invention is not limited to the above-described embodiments. The embodiments are just examples, and any embodiments that substantially have the same feature and demonstrate the same functions and effects as those in the technical concept disclosed in claims of the present invention are included in the technical scope of the present invention.

## Claims

1. A cosmetic comprising at least one organosiloxane represented by the following general formula (1), and having a boiling point in a range of 205 to 255°C and a viscosity of less than 5 mm²/s at 25°C, 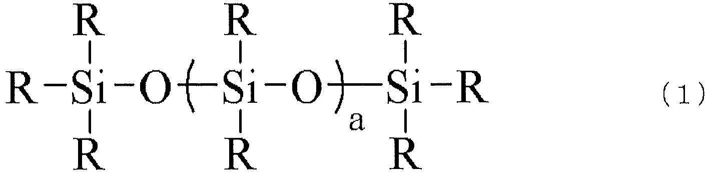 wherein R's are identical to or different from one another and each independently represent a hydrogen group, a hydroxy group, or a monovalent hydrocarbon group having 1 to 3 carbon atoms, but at least one R is a monovalent hydrocarbon group having 2 to 3 carbon atoms; and "a" represents 1 to 5.

2. The cosmetic according to claim 1, wherein in the general formula (1) of the organosiloxane, "a" is 2.

3. The cosmetic according to claim 1 or 2, wherein the organosiloxane is selected from the group consisting of 3,5-diethyl-1,1,1,3,5,7,7,7-octamethyltetrasiloxane, 3,3-diethyl-1,1,1,5,5,7,7,7-octamethyltetrasiloxane, 1,3-diethyl-1,1,3,5,5,7,7,7-octamethyltetrasiloxane, 1,5-diethyl-1,1,3,3,5,7,7,7-octamethyltetrasiloxane, 1,7-diethyl-1,1,3,3,5,5,7,7-octamethyltetrasiloxane, 3,5-dipropyl-1,1,1,3,5,7,7,7-octamethyltetrasiloxane, 3,3-dipropyl-1,1,1,5,5,7,7,7-octamethyltetrasiloxane, 1,3-dipropyl-1,1,3,5,5,7,7,7-octamethyltetrasiloxane, 1,5-dipropyl-1,1,3,3,5,7,7,7-octamethyltetrasiloxane, 1,7-dipropyl-1,1,3,3,5,5,7,7-octamethyltetrasiloxane, and 3-ethyl-5-propyl-1,1,1,3,5,7,7,7-octamethyltetrasiloxane.

4. The cosmetic according to any one of claims 1 to 3, further comprising an organic ultraviolet absorber.

5. The cosmetic according to claim 4, wherein the organic ultraviolet absorber comprises one or more selected from ethylhexyl methoxycinnamate, diethylamino hydroxybenzoyl hexyl benzoate, octyl salicylate, polysilicone-15, t-butyl methoxydibenzoylmethane, oxybenzone, methylene bis-benzotriazolyl tetramethylbutylphenol, bis-ethylhexyloxyphenol methoxyphenyl triazine, and octocrylene.

6. The cosmetic according to any one of claims 1 to 5, further comprising an oily component being solid at 25°C.

7. The cosmetic according to claim 6, wherein the oily component being solid at 25°C comprises one or more selected from polyethylene, ceresin, ozokerite, beeswax, microcrystalline wax, stearyl alcohol, behenyl alcohol, and cetanol.
